# EUROPEAN PATENT APPLICATION

(11) **EP 1 797 900 A1**
(43) Date of publication of application: **20.06.2007**
(21) Application number: 05710502.5
(22) Date of filing: 22.02.2005
(51) Int. Cl.: A61K 47/02

(54) **PREPARATION FOR PERCUTANEOUS/PERMUCOSAL ABSORPTION**

(30) Priority: 07.10.2004 JP 2004294740
(71) Applicant: KABUSHIKI KAISHA SANGI, Tokyo 104-0045 (JP)
(72) Inventor: SAKUMA, Shuji, Chuo-ku, Tokyo 1040045 (JP); ATSUMI, Kiminori, Chuo-ku, Tokyo 1040045 (JP); KIKUKAWA, Keiichiro, Chuo-ku, Tokyo 1040045 (JP)
(74) Representative: Vossius & Partner
(86) International application number: PCT/JP2005/002771
(87) International publication number: WO 2006/038315

(57) **Abstract**

Transdermal and transmucosal preparations are often insufficiently absorbed. Thus, there is a need for additives that can enhance the transdermal and/or transmucosal absorption of various drugs. The present invention provides compositions for enhancing transdermal and/or transmucosal absorption, comprising a hydroxyapatite and essential ingredients. The hydroxyapatite has a maximum particle size of 1 µm or less, preferably 0.1 µm or less, and the content of the hydroxyapatite relative to a drug to be formulated ranges from 0.1 to 1000 weight percent.

## Description

### Technical Field

The present invention relates to transdermal and transmucosal preparationsthat are applied to the skin or mucosa, and which enhance the transdermal and/or transmucosal absorption of essential ingredients.

### Background Art

Major dosage forms that have been used for administering drugs to the body's circulatory system are oral form, injectable form, and so on. In recent years, many drugs have utilized transdermal and transmucosal preparations, which are applied to the skin and/or mucosa such as oral, nasal, rectal, vaginal, and ophthalmic mucosa, via transdermal or transmucosal absorption. Such preparations are advantageous and convenient for pharmaceutical use because the blood concentrations of which are easily maintained at or above a certain level for long periods of time, and the administration of which can be easily discontinued when necessary. In addition, they are not painful like injections and their drug efficacy is hardly reduced because of the gastrointestinal tract environment or liver metabolism.

However, transdermal/transmucosal drug administrations generally have low efficiencies of drug absorption via the skin's horny layer or mucosa, which functions as a barrier to prevent invasion by foreign substances, and thus it would be hard to administer drugs at their therapeutically effective doses.

Thus, various methods and compositions for enhancing the transdermal / transmucosal absorption have been disclosed: methods which enhance absorption by mixing drugs with geranyl acetate to impair the barrier function of the epidermal cell layers (Japanese Patent Application Kokai Publication No. (JP-A) H5-178762 (unexamined, published Japanese patent application)); dextran-based pharmaceutical carriers that be absorbed by mucosa (JP-A H5-238956); transdermal patches laminated with an adhesive layer consisting of an adhesive, a tackifier, a drug, a transdermal absorption enhancer, and a release stimulator for the transdermal absorption enhancer (JP-A H7-101864); external preparations comprising 10 to 20 weight percent glycerin monooleate, 30 to 60 weight percent ethanol, and 30 to 60 weight percent water for enhancing the percutaneous absorption of pharmaceutically active substances ((Granted) Japanese Patent No. 3471840); microemulsion preparations for improving transdermal/transmucosal absorption (JP-A H7-2689); transdermal absorption enhancers comprising glyceryl-denatured silicone ((Granted) Japanese Patent No. 3417744); transmucosal preparation compositions comprising poorly absorbable substances and arginine, a polymer thereof, or a salt of the polymer (JP-A H10-95738); external preparations containing gritty particles to enhance the transdermal absorption and/or the transmucosal absorption of drugs (JP-A H11-80031); methods for enhancing transdermal drug absorptions by combining 4 to 15 µm ceramics with external patches (Rinsho Yakuri (Clinical Pharmacology and Therapeutics) 23(1) Mar. 1992); transdermal absorption enhancers comprising as an active ingredient an oil solution having solubility parameters within a certain range; transdermal absorption enhancers comprising a labiate plant extract; transdermal absorption enhancers comprising as an active ingredient a polyhydric alcohol fatty acid ester or a sulfuric acid alkyl ester; transmucosal absorption enhancers comprising middle-chain fatty acid salts and glycyrrhetinic acid salts; transmucosal absorption-enhancing agents comprising arginine, an arginine polymer, an arginine salt, or such; transdermal absorption-enhancing compositions comprising propylene glycol, polyol fatty acid ester, or Lauromacrogol; methods for increasing drug concentrations, and so on. A method which uses a pharmaceutical agent mixed with fine crystals of calcium phosphate as an intravascular hydroxyapatite injection has also been disclosed. In this method, intravascular administration of a pharmaceutical agent enhances or delays the effect of the pharmaceutical agent, and allows the pharmaceutical agent to selectively attach to various cells such as cancer cells and viruses such as AIDS, ATL and hepatitis viruses. The differentiation and growth of these cancer cells or viruses are thereby regulated while the effect of the pharmaceutical agent is maintained (JP-A H5-255095).

However, transdermal and transmucosal preparations are often not well absorbed, and thus there is a need to develop additives which enhance the transdermal and transmucosal absorptions for various pharmaceutical agents.
Patent Document 1: JP-A H5-178762
Patent Document 2: JP-A H5-238956
Patent Document 3: JP-A H7-101864
Patent Document 4: (Granted) Japanese Patent No. 3471840
Patent Document 5: JP-A H7-2689
Patent Document 6: (Granted) Japanese Patent No. 3417744
Patent Document 7: JP-A H10-95738
Patent Document 8: JP-A H11-80031
Patent Document 9: JP-A H5-255095
Non-patent Document 1: Rinsho Yakuri (Clinical Pharmacology and Therapeutics) 23(1), Mar. 1992

### Disclosure of the Invention

An objective of the present invention is to provide transdermal and transmucosal preparations having an excellent effect of enhancing the transdermal and/or transmucosal absorption of essential ingredients when applied to the skin or mucosa.

The present inventors conducted exhaustive studies to achieve the objective described above. As a result, the inventors discovered that the transdermal and transmucosal absorptions of essential ingredients can be enhanced by applying the transdermal or transmucosal composition comprising the essential ingredients and hydroxyapatite to the skin or mucosa, and thus completed the present invention.

Hydroxyapatites for use in the present invention typically have a stoichiometric composition of Ca₁₀ (PO₄)₆ (OH)₂ , and have the characteristics of a hydroxyapatite as well as an apatite structure, even when they are non-stoichiometric compositions having a non-1.67 Ca/P molar ratio.

Hydroxyapatites, either stoichiometric or non-stoichiometric, that have a Ca/P molar ratio within the range of 1.4 to 1.8 can be used in the present invention.

The Ca/P molar ratios of hydroxyapatites can be controlled by altering the mixing ratio of salt ingredients and the synthesis condition. For example, in wet hydroxyapatite synthesis methods, the Ca/P molar ratio can be increased by alkalizing an aqueous solution with ammonia water or such during synthesis. Alternatively, the Ca/P molar ratio can be reduced by neutralizing or weakly acidifying the aqueous solution with a diluted acid.

The above-mentioned hydroxyapatites for use in the present invention include both crystalline and poorly crystalline hydroxyapatites. Poorly crystalline or amorphous hydroxyapatites are preferable.

Herein, the term "poorly crystalline" refers to crystalline powders showing a broader peak than a highly crystalline powder in X-ray diffraction patterns.

The term "amorphous" refers to powders comprising fine particles, whose X-ray diffraction pattern exhibits a broad halo and is uncharacteristic of crystals. Hereinafter, a poorly crystalline hydroxyapatite and an amorphous hydroxyapatite are referred to as a "poorly crystalline apatite" and an "amorphous apatite", respectively.

The poorly crystalline apatites and amorphous apatites for use in the present invention include, for example, apatites that have been prepared by the above-mentioned wet synthesis method and then freeze-dried, or dried at a lower-than-100°C temperature, or baked at a temperature lower than 300°C.

The poorly crystalline apatites and amorphous apatites comprise particles whose size is less than that of highly crystalline hydroxyapatites (hereinafter referred to as "crystalline apatites").

In the present invention, maximum particle size of a hydroxyapatite is preferably 1.0 µm or less, more preferably 0.1 µm or less, and still more preferably 0.05 µm or less.

Mean particle size is preferably 0.6 µm or less, more preferably 0.07 µm or less, and still more preferably 0.04 µm or less.

As the mean particle size gets smaller, the specific surface becomes larger, leading to an increased absorption of pharmaceutical agent. Accordingly, the mean particle size does not have a particularly limited lower limit; however, it is preferably, for example, about 0.01 µm.

The maximum hydroxyapatite particle size can be adjusted by crushing to a particle size of preferably 1.0 µm or less, more preferably 0.1 µm or less, and still more preferably 0.05 µm or less. After being crushed to fine particles with a maximum size of 1.0 µm or less, preferably 0.1 µm or less, and more preferably 0.05 µm or less, the hydroxyapatite may be combined with essential ingredients, as well as other ingredients and bases for use in transdermal/transmucosal preparations. To enhance transdermal and transmucosal absorption, however, it is more preferable that the essential ingredients are first carried by crushed hydroxyapatite, followed by combining the mixture with other ingredients and bases to be used in transdermal/transmucosal preparations.

The hydroxyapatite content in transdermal/transmucosal preparations varies depending on the essential ingredients, and thus it is difficult to have a fixed quantity of hydroxyapatite for all preparations. The content preferably ranges from 0.1 to 1000 weight percent based on the essential ingredients. To achieve a sufficient transdermal or transmucosal absorption effect, it is more preferable that the content ranges from 1 to 500 weight percent, and still more preferably from 10 to 200 weight percent.

The skin or mucosa permeability of essential ingredients in various transdermal/transmucosal preparations can be markedly enhanced when the transdermal/transmucosal preparations comprise a hydroxyapatite of the present invention.

In the present invention, the type of ingredients to be absorbed through the skin or mucosa is not particularly limited. Such ingredients include various pharmaceutical agents and cosmetic ingredients, as long as they can be used for transdermal and/or transmucosal absorption.

Specifically, such ingredients include, but are not limited to: analgesic, antipyretic, or anti-inflammatory agents, antigout or antihyperuricemic agents, nonsteroidal anti-inflammatory agents, steroidal anti-inflammatory agents, wound healing agents, anticancer agents, hypnotic or analgesic agents, anxiolytic agents, antipsychotic agents, antidepressant agents, antimanic agents, antihistaminic agents, antiepileptic agents, local anesthetics, therapeutic agents for peripheral circulation disorders, antiparkinson agents, muscle relaxants, autonomic or antispasmodic agents, antidiaphoretic agents, cerebral circulation or metabolism-improving drugs, cardiac stimulants, antianginal agents, β blockers, Ca antagonists, antiarrhythmic agents, antihypertensive agents, vasodilators, decongestants, capillary stabilizers, antihyperlipemic agents, vasopressors, respiratory stimulants, bronchodilators or antiasthmatic agents, antitussive agents, expectorants, peptic ulcer agents, deglutitory agents, antiemetic agents, obstipants or antiflatulents, stomachics or digestants, cathartics, hemorrhoidal preparations, therapeutics for neurogenic bladder, therapeutics for benign prostatic hyperplasia or frequent urination, therapeutics for nephrolithiasis or urolithiasis, diuretic agents, choleretic agents, antidiabetic agents, pituitary hormone preparations, adrenocortical hormone preparations, sex hormone preparations, prostaglandins, therapeutic agents for thyroid dysfunction, anti-osteoporosis or bone metabolism improving drugs, amino acids, vitamin preparations, hemostatic agents, antithrombotic agents, antibiotics, sulfa drugs, antimycotic agents, antiviral agents, anti-HIV agents, antiparasitic or antiprotozoal agents, antirheumatic drugs, immunosuppressants, sunscreen agents, antiallergic agents, ophthalmic preparations, antipsoriatic agents, skin softeners or emollients, moisturizing ingredients, baldness-remedies or hair growth stimulants, peptide hormone preparations, anti-smoking aids, diet ingredients, skin whitening ingredients, anti-aging ingredients, polysaccharides, plant extracts or essential oils, enzymes, and vaccines. They may be used singly or in combination.

In another embodiment, the present invention provides uses of hydroxyapatites and ingredients to be absorbed transdermally and/or transmucosally to produce transdermal and transmucosal preparations. Hydroxyapatites with the above-mentioned particle sizes can be preferably used.

The methods for preventing or treating diseases of the present invention comprise the step of administering an effective dose of a transdermal or transmucosal preparation comprising an above-mentioned hydroxyapatite and ingredient to be absorbed transdermally or transmucosally. Furthermore, the methods of the present invention for promoting beauty and health comprise the step of administering effective doses of the transdermal and/or transmucosal preparation described above.

The "ingredients to be absorbed transdermally and/or transmucosally" that can be used in the present invention are described below in detail, but are not limited thereto. Diseases to be targeted by the present invention's preventive or therapeutic methods, or by the present invention's methods for promoting beauty and health are diseases that respond to the ingredients described above.

### [Analgesic, antipyretic, and anti-inflammatory agents]

Aspirin, acetaminophen, ibuprofen, indomethacin, ketoprofen, diclofenac sodium, diflunisal, piroxicam, phenacetin, fenoprofen calcium, felbinac, flurbiprofen, chlorpheniramine maleate, salicylic acid, methyl salicylate, etc.

### [Antigout and antihyperuricemic agents]

Colchicine, benzbromarone, probenecid, sulfinpyrazone, allopurinol, etc.

### [Nonsteroidal anti-inflammatory agents]

Aspirin, acetaminophen, ibuprofen, indomethacin, etodolac, salicylic acid, salicylate glycol, diclofenac sodium, diflunisal, suprofen, sulindac, ketoprofen, tolmetin sodium, flurbiprofen, fenoprofen, fenoprofen calcium, zaltoprofen, piroxicam, aminopyrine, antipyrine, sulpyrine, oxyphenbutazone, naproxen, etofenamate, salicylamide, sulindac, triethanol aminosalicylate, mefenamic acid, flufenamic acid, meclofenamic acid, colchicine, bufexamac, felbinac, loxoprofen, fenbufen, diflunisal, alclofenac, phenylbutazone, bendazac, etc.

### [Steroidal anti-inflammatory agents]

Amcinonide, prednisolone valerate, diflucortolone valerate, betamethasone valerate, betamethasone acetate, dexamethasone acetate, betamethasone dipropionate, dexamethasone, flumethasone pivalate, beclomethasone propionate, betamethasone, triamcinolone acetonide, halcinonide, fluocinonide, fluocinolone acetonide, fluorometholone, fludroxycortide, prednisolone, methylprednisolone, methylprednisolone acetate, hydrocortisone, hydrocortisone butyrate, clobetasol propionate, etc.

### [Wound healing-enhancing agents]

Allantoin and derivatives thereof, glycyrrhizin acid and derivatives thereof, glycyrrhetic acid and derivatives thereof, ε-aminocaproic acid, *Glycyrrhiza Radix, Lithospermum Radix,* azulene, sodium azulene sulfonate, berberine chloride, berberine sulfate, lysozyme chloride, hinokitiol, aloe, etc.

### [Anticancer agents]

Cyclophosphamide, 5-fluorouracil, idarubicin hydrochloride, epirubicin hydrochloride, bleomycin hydrochloride, daunorubicin hydrochloride, doxorubicin hydrochloride, pirarubicin hydrochloride, actinomycin C, adriamycin, mitomycin C, gefitinib, interferon β, Picibanil, 6-mercaptopurine, etoposide, cisplatin, carboplatin, nedaplatin, oxaliplatin, carboquone, zinostatin stimalamer, doxorubicin, nimstine hydrochloride, neocarzinostatin, paclitaxel, melphalan, tretinoin, estramustine sodium phosphate, daunorubicin, hydroxycarbamide, tegafur, mercaptopurine, L-asparaginase, methotrexate, sobuzoxane, busulfan, tamoxifen citrate, flutamide, UFT, peplomycin sulfate, vinblastine sulfate, vincristine, vincristine sulfate, cytarabine ocfosfate, doxifluridine, chromomycin A, aceglatone, anastrozole, ubenimex, fadrozole hydrochloride hydrate, procarbazine hydrochloride, toremifene citrate, bicalutamide, imatinib mesylate, etc.

### [Hypnotic and analgesic agents]

Bromvalerylurea, allyl isopropyl acetyl urea, estazolam, triazolam, midazolam, flunitrazepam, nimetazepam, nitrazepam, flurazepam, haloxazolam, quazepam, zopiclone, zolpidem, hexobarbital, pentobarbital, amobarbital, barbital, butoctamide semisuccinate, chloral hydrate, bromvalerylurea, sodium trichloroethyl, perlapine, morphine, morphine sulfate, hydromorphine, oxymorphone, codeine, dihydrocodeine, naloxone, nalorphine, pentazocine, methadone, methadone hydrochloride, brotizolam, haloxazolam, phenobarbital, rilmazafone hydrochloride, etc.

### [Anxiolytic agents]

Bromvalerylurea, amobarbital, diazepam, nitrazepam, flurazepam, clotiazepam, flutoprazepam, lorazepam, prazepam, medazepam, bromazepam, oxazepam, tofisopam, dipotassium clorazepate, fludiazepam, alprazolam, etizolam, oxazolam, cloxazolam, mexazolam, flutazolam, hydroxyzine, hydroxyzine hydrochloride, thioridazine hydrochloride, chlordiazepoxide, pyrantel pamoate, chlorpromazine hydrochloride, levomepromazine maleate, ethyl loflazepate, etc.

### [Antipsychotic agents]

Amoxapine, amitriptyline hydrochloride, nortriptyline hydrochloride, maprotiline hydrochloride, imipramine hydrochloride, clocapramine, chlorpromazine, sulpiride, thioridazine, timiperone, trifluoperazine, nemonapride, haloperidol, fluphenazine enanthate, fluphenazine decanoate, decanoate haloperidol, zotepine, risperidone, quetiapine fumarate, olanzapine, trimipramine maleate, levomepromazine maleate, etc.

### [Antidepressant agents]

Amoxapine, amitriptyline hydrochloride, imipramine hydrochloride, clomipramine hydrochloride, safrazine hydrochloride, lofepramine hydrochloride, dosulepin hydrochloride, trazodone hydrochloride, nortriptyline hydrochloride, maprotiline hydrochloride, mianserin hydrochloride, milnacipran hydrochloride, trimipramine maleate, setiptiline maleate, fluvoxamine maleate, etc.

### [Antimanic agents]

Lithium carbonate, etc.

### [Antihistaminic agents]

Diphenhydramine, diphenhydramine hydrochloride, diphenhydramine tannate, diphenhydramine lauryl sulfate, chlorpheniramine maleate, alimemazine tartrate, triprolidine hydrochloride, meclizine hydrochloride, promethazine, promethazine hydrochloride, homochlorcyclizine hydrochloride, mequitazine, clemastine fumarate, iproheptine hydrochloride, cyproheptadine hydrochloride, diphenylpyraline teoclate, mebhydrolin napadisylate, diphenylpyraline hydrochloride, dimenhydrinate, isothipendyl hydrochloride, dimethindene maleate, etc.

### [Antiepileptic agents]

Phenobarbital, phenobarbital sodium, metharbital, primidone, Hydantol D, E, and F, carbamazepine, sodium valproate, ethotoin, phenytoin, trimethadione, acetylpheneturide, sultiame, clonazepam, diazepam, nitrazepam, ethosuximide, zonisamide, acetazolamide, ACTH, vitamin B₆, etc.

### [Local anesthetics]

Cocaine, ethyl aminobenzoate, tetracaine hydrochloride, procaine, procaine hydrochloride, dibucaine, dibucaine hydrochloride, lidocaine, lidocaine hydrochloride, oxybuprocaine hydrochloride, bupivacaine hydrochloride, T-CAIN, mepivacaine hydrochloride, propitocaine hydrochloride, oxethazaine, etc.

### [Therapeutic agents for peripheral circulation disorders]

Inositol hexanicotinate, hepronicate, tolazoline hydrochloride, isoxsuprine hydrochloride, etc.

### [Antiparkinson agents]

Levodopa, droxidopa, pergolide mesylate, bromocriptine mesylate, trihexyphenidyl hydrochloride, talipexole hydrochloride, amantadine hydrochloride, biperiden hydrochloride, piroheptine hydrochloride, profenamine hydrochloride, mazaticol hydrochloride, methixene hydrochloride, etc.

### [Muscle relaxants]

Diazepam, tubocurarine chloride, suxamethonium chloride, pancuronium bromide, vecuronium bromide, dantrolene sodium, afloqualone, eperisone hydrochloride, tolperisone hydrochloride, chlorphenesin carbamate, baclofen, tizanidine hydrochloride, pridinol mesylate, phenprobamate, methocarbamol, etc.

### [Autonomic and antispasmodic agents]

Bethanechol chloride, carpronium chloride, acetylcholine chloride, neostigmine, pyridostigmine bromide, distigmine bromide, ambenonium chloride, edrophonium chloride, prifinium bromide, glycopyrronium bromide, atropine sulfate, anisotropine methylbromide, scopolamine hydrobromate, butyl scopolamine bromide, N-methyl scopolamine methyl sulfate, papaverine hydrochloride, oxapium iodide, valethamate bromide, piperidolate hydrochloride, scopolia extract, butropium bromide, trepibutone, etomidoline, timepidium bromide, tiquizium bromide, ethyl pipethanate bromide, tiemonium iodide, methylbenactyzium bromide, propantheline bromide, dicyclomine hydrochloride, flopropione, ergometrine maleate, tofisopam, etc.

### [Antidiaphoretic agents]

Scopolamine, propantheline bromide, quaternary alloxy methyl ammonium salts, etc.

### [Cerebral circulation and metabolism-improving drugs]

Citicoline, adenosine triphosphate disodium, γ-amino butyrate, γ-amino- β-hydroxybutyrate, γ-orizanol, calcium hopantenate, idebenone, meclofenoxate hydrochloride, bifemelane hydrochloride, indeloxazine hydrochloride, lisuride maleate, aniracetam, tiapride hydrochloride, ifenprodil tartrate, fasudil hydrochloride, nicergoline, ibudilast, brovincamine fumarate, pentoxifylline, flunarizine hydrochloride, moxisylyte hydrochloride, dihydroergotoxine mesylate, ozagrel sodium, nizofenone fumarate, etc.

### [Cardiac stimulants]

Digoxin, digitoxin, methyldigoxin, lanatoside C, deslanoside, proscillaridin, dopamine hydrochloride, dobutamine hydrochloride, docarpamine, epinephrine, norepinephrine, isoprenaline hydrochloride, amrinone, olprinone hydrochloride, milrinone, carperitide, aminophylline, diprophylline, vesnarinone, pimobendan, crataegus extract, trans-π-oxocamphor, ubidecarenone, aminoethyl sulfonate, carperitide, etc.

### [Antianginal agents]

Nitroglycerin, amyl nitrite, isosorbide nitrate, isosorbide mononitrate, isosorbide dinitrate, nifedipine, nicardipine hydrochloride, nilvadipine, nisoldipine, nitrendipine, manidipine hydrochloride, benidipine hydrochloride, barnidipine hydrochloride, amlodipine besilate, efonidipine hydrochloride, felodipine, cilnidipine, verapamil hydrochloride, diltiazem hydrochloride, etafenone hydrochloride, dipyridamole, trapidil, nicorandil, trimetazidine hydrochloride, dilazep hydrochloride, etc.

### [β blockers]

Propranolol hydrochloride, bufetolol hydrochloride, bupranolol hydrochloride, alprenolol hydrochloride, indenolol hydrochloride, oxprenolol hydrochloride, bunitrolol hydrochloride, penbutolol sulfate, bopindolol malonate, pindolol, carteolol hydrochloride, metoprolol tartrate, atenolol, bisoprolol fumarate, betaxolol hydrochloride, acebutolol hydrochloride, celiprolol hydrochloride, labetalol hydrochloride, carvedilol, bevantolol hydrochloride, amosulalol hydrochloride, arotinolol hydrochloride, nipradilol, tilisolol hydrochloride, etc.

### [Ca antagonists]

Nifedipine, nicardipine hydrochloride, nilvadipine, nisoldipine, nitrendipine, manidipine hydrochloride, benidipine hydrochloride, barnidipine hydrochloride, efonidipine hydrochloride, felodipine, cilnidipine, aranidipine, amlodipine besilate, verapamil hydrochloride, diltiazem hydrochloride, etc.

### [Antiarrhythmic agents]

Oxprenolol hydrochloride, quinidine sulfate, procaineamide hydrochloride, disopyramide, disopyramide phosphate, ajmaline, cibenzoline succinate, bepridil hydrochloride, pirmenol hydrochloride, propranolol hydrochloride, lidocaine hydrochloride, nifekalant hydrochloride, sotalol hydrochloride, mexiletine hydrochloride, aprindine hydrochloride, flecainide acetate, pilsicainide hydrochloride, propafenone hydrochloride, amiodarone hydrochloride, bretylium, bufetolol hydrochloride, bupranolol hydrochloride, bucumolol hydrochloride, timolol maleate, nadolol, pindolol, bopindolol malonate, alprenolol hydrochloride, indenolol hydrochloride, carteolol hydrochloride, penbutolol sulfate, bunitrolol hydrochloride, metoprolol tartrate, acebutolol, atenolol, bisoprolol fumarate, betaxolol hydrochloride, celiprolol hydrochloride, labetalol hydrochloride, arotinolol hydrochloride, amosulalol hydrochloride, carvedilol, nipradilol, tilisolol hydrochloride, bevantolol hydrochloride, etc.

### [Antihypertensive agents]

Methyldopa, guanabenz acetate, reserpine, rescinnamine, guanfacine hydrochloride, guanfacine sulfate, prazosin hydrochloride, bunazosin hydrochloride, terazosin hydrochloride, urapidil, doxazosin mesylate, dihydroergotoxine mesylate, prazosin, alacepril, captopril, enalapril maleate, delapril hydrochloride, cilazapril, lisinopril, benazepril hydrochloride, imidapril hydrochloride, quinapril hydrochloride, temocapril hydrochloride, trandolapril, clonidine, clonidine hydrochloride, guanethidine, betanidine, guanethidine sulfate, hydralazine hydrochloride, budralazine, todralazine hydrochloride, cadralazine, sodium nitroprusside, hexamethonium bromide, trimethaphan camsylate, meticrane, methyldopa, etc.

### [Vasodilators]

Efloxate, etafenone, oxyfedrine, carbocromen, dilazep dihydrochloride, diltiazem hydrochloride, benidipine hydrochloride, verapamil hydrochloride, nisoldipine, nitrendipine, amlodipine besilate, pentaerythritol tetranitrate, nicorandil, dipyridamole, isosorbide nitrate, trapidil, nitroglycerin, nifedipine, prenylamine, molsidomine, trolnitrate phosphate, inositol hexanicotinate, isoxsuprine, nylidrin, nicametate citrate, nicotinic alcohol, cyclandelate, hepronicate, trimetazidine hydrochloride, cinnarizine, budralazine, hydralazine hydrochloride, todralazine hydrochloride, cadralazine, sodium nitroprusside, isoxsuprine hydrochloride, bamethan sulfate, etc.

### [Decongestants]

Epinephrine, epinephrine hydrochloride, ephedrine hydrochloride, tetrahydrozoline hydrochloride, naphazoline hydrochloride, etc.

### [Capillary stabilizers]

Rutin, etc.

### [Antihyperlipemic agents]

Clofibrate, clinofibrate, simfibrate, bezafibrate, fenofibrate, nicomol, niceritrol, tocopherol nicotinate, colestyramine, probucol, pravastatin sodium, simvastatin, fluvastatin sodium, atorvastatin calcium hydrate, aluminium clofibrate, soybean oil unsaponifiables, purified soybean lecithin, γ-orizanol, oxymetholone, ethyl linoleate, dextran sodium sulfate, polyenephosphatidylcholine, estarase, ethyl icosapentate, colestimide, melinamide, etc.

### [Vasopressors]

Dopamine hydrochloride, dobutamine hydrochloride, docarpamine, denopamine, phenylephrine hydrochloride, epinephrine, norepinephrine, norphenephrine hydrochloride, etilefrine hydrochloride, isoprenaline hydrochloride, midodrine hydrochloride, bucladesine sodium, metaraminol bitartrate, methoxamine hydrochloride, amezinium metilsulfate, etc.

### [Respiratory stimulants]

Doxapram hydrochloride, medroxyprogesterone, levallorphan, naloxone hydrochloride, theophylline, diprophylline, dimefline hydrochloride, isoprenaline hydrochloride, orciprenaline sulfate, clorprenaline hydrochloride, terbutaline sulfate, hexoprenaline sulfate, ephedrine hydrochloride, methylephedrine hydrochloride, dl-methylephedrine saccharinate, methoxyphenamine hydrochloride, dimorphoramine, trimethoxynol hydrochloride, salbutamol sulfate, formoterol fumarate, tulobuterol hydrochloride, tulobuterol, fenoterol hydrobromide, procaterol hydrochloride, clenbuterol hydrochloride, mabuterol hydrochloride, isoproterol sulfate, digoxin, digitoxin, methyldigoxin, lanatoside C, deslanoside, proscillaridin, etc.

### [Bronchodilators and antiasthmatic agents]

Theophylline, aminophylline, choline theophylline, diprophylline, ephedrine hydrochloride, methylephedrine hydrochloride, dl-methylephedrine saccharinate, methoxyphenamine hydrochloride, orciprenaline sulfate, clorprenaline hydrochloride, trimethoxynol hydrochloride, salbutamol sulfate, terbutaline sulfate, hexoprenaline sulfate, isoprenaline hydrochloride, formoterol fumarate, tulobuterol, tulobuterol hydrochloride, fenoterol hydrobromide, procaterol hydrochloride, clenbuterol hydrochloride, mabuterol hydrochloride, salbutamol, terbutaline, tulobuterol, fenoterol, procaterol, clenbuterol, isoproterol sulfate, sodium cromoglicate, tranilast, pemirolast, ketotifen fumarate, azelastine, beclomethasone propionate, ipratropium bromide, flutropium bromide, oxitropium bromide, nifedipine, nitrendipine, manidipine hydrochloride, benidipine hydrochloride, barnidipine hydrochloride, amlodipine besilate, efonidipine hydrochloride, felodipine, cilnidipine, verapamil hydrochloride, diltiazem hydrochloride, etc.

### [Antitussive agents]

Noscapine, dimemorfan phosphate, tipepidine hibenzate, oxeladin citrate, guaifenesin, dextromethorphan hydrobromate, pentoxyverine citrate, eprazinone hydrochloride, fominoben hydrochloride, chloperastine, clofedanol hydrochloride, benproperine phosphate, noscapine, cherry bark extract, plantago herb, apricot kernel, etc.

### [Expectorants]

L-methylcysteine hydrochloride, acetylcysteine, bromhexine hydrochloride, carbocysteine, fudosteine, ambroxol hydrochloride, foeniculated ammonia spirit, senega, etc.

### [Therapeutic agents for peptic ulcer]

Cimetidine, ranitidine hydrochloride, famotidine, omeprazole, omeprazole sodium, lansoprazole, rabeprazole sodium, roxatidine acetate hydrochloride, nizatidine, lafutidine, pirenzepine hydrochloride, proglumide, secretin, urogastrone, magnesium oxide, precipitated calcium carbonate, dried aluminum hydroxide gel, magnesium aluminometasilicate, synthetic aluminum silicate, hydrotalcite, sucralfate, azulene, egualen sodium, aldioxa, gefarnate, teprenone, ornoprostil, cetraxate hydrochloride, sulpiride, irsogladine maleate, etc.

### [Deglutitory agents]

Magnesium sulfate, sodium sulfate, magnesium citrate, artificial carlsbad salt, carmellose sodium, castor oil, bisacodyl, sodium picosulfate, phenovalin, senna extract, sennoside, etc.

### [Antiemetic agents]

Chlorpromazine, etc.

### [Obstipants and antiflatulents]

Loperamide hydrochloride, bismuth subnitrate, albumin tannate, berberine chloride, berberine sulfate, berberine tannate, natural aluminum silicate, tilactase, β-galactosidase, mepenzolate bromide, polycarbophil calcium, *Mallotus japonicus,* salazosulfapyridine, mesalazine, *Lactobacillus bifidus, Lactobacillus casei, Clostridium butyricum,* lactomin, antibiotics-resistant lactic acid bacteria, dried yeast, dimeticone, etc.

### [Stomachics and digestants]

Metoclopramide, domperidone, cisapride, itopride hydrochloride, mosapride citrate, trimebutine maleate, diastase, pancreatin, etc.

### [Cathartic agents]

Carmellose sodium, castor oil, bisacodyl, sodium picosulfate, phenovalin, senna extract, sennoside, etc.

### [Hemorrhoidal preparations]

Tribenoside, Hemolingual, melilot extract, Circanetten, phenol, etc.

### [Therapeutic agents for neurogenic bladder]

Oxybutynin hydrochloride, propiverine hydrochloride, flavoxate hydrochloride, etc.

### [Therapeutic agents for benign prostatic hyperplasia and frequent urination]

Oxendolone, gestonorone caproate, chlormadinone acetate, Paraprost, Eviprostat, allylestrenol, tamsulosin hydrochloride, Naftopidil, cernitin pollen extract, etc.

### [Therapeutic agents for nephrolithiasis and urolithiasis]

*Quercus salicina* extract, etc.

### [Diuretic agents]

Trichlormethiazide, hydrochlorothiazide, benzylhydrochlorothiazide, indapamide, tripamide, chlortalidone, meticrane, mefruside, furosemide, bumetanid, ethacrynic acid, piretanide, azosemide, torasemide, spironolactone, triamterene, potassium canrenoate, acetazolamide, isosorbide, etc.

### [Choleretic agents]

Hymecromone, anetholtrithion, osalmide, etc.

### [Antidiabetic agents]

Insulin, tolbutamide, chlorpropamide, acetohexamide, glyclopyramide, tolazamide, glibenclamide, gliclazide, glimepiride, glybuzole, buformin hydrochloride, metformin hydrochloride, pioglitazone hydrochloride, nateglinide, acarbose, voglibose, epalrestat, etc.

### [Pituitary hormone preparations]

Somatropin, mecasermin, tetracosactide acetate, pituitary gonadotropin, serum gonadotropin, placental gonadotropin, vasopressin, desmopressin acetate, oxytocin, etc.

### [Adrenocortical hormone preparations]

Cortisone acetate, hydrocortisone, hydrocortisone acetate, hydrocortisone sodium phosphate, hydrocortisone sodium succinate, fludrocortisone acetate, prednisolone, prednisolone acetate, prednisolone butyl acetate, prednisolone sodium succinate, prednisolone sodium phosphate, halopredone acetate, methylprednisolone, methylprednisolone acetate, methyl prednisolone sodium succinate, triamcinolone, triamcinolone acetate, triamcinolone acetonide, dexamethasone, dexamethasone acetate, dexamethasone sodium phosphate, dexamethasone palmitate, mixture of betamethasone and betamethasone phosphate, betamethasone, betamethasone sodium phosphate, paramethasone acetate, beclomethasone propionate, etc.

### [Sex hormone preparations]

Estradiol, 17β-estradiol, estradiol benzoate, estradiol propionate, estradiol valerate, ethynylestradiol, estriol, estriol propionate, estriol acetate benzoate, conjugated estrogen, fosfestrol, progesterone, nortestosterone, gestonorone caproate, norethisterone, norethisterone acetate, pregnanediol, testosterone propionate, testosterone enanthate, kallidinogenase, sodium prasterone sulfate, alprostadil, beraprost sodium, epoprostenol sodium, gemeprost, dinoprost, dinoprost tromethamine, dinoprostone betadex, cyproterone acetate, methyltestosterone, fluoxymesterone, dromostanolone propionate, clomiphene citrate, cyclofenil, danazol, goserelin acetate, buserelin acetate, nafarelin acetate, bromocriptine mesilate, terguride, etc.

### [Prostaglandin preparations]

Prostaglandin F₂α, prostacyclin, prostaglandin E₁, prostaglandin E₂, alprostadil, beraprost sodium, gemeprost, dinoprostone, dinoprost, dinoprost tromethamine, 5-fluoroprostacyclin, 7-fluoroprostacyclin, etc.

### [Therapeutic agents for thyroid dysfunction]

Levothyroxine sodium, liothyronine sodium, dried thyroid, thiamazole, propylthiouracil, etc.

### [Anti-osteoporosis and bone metabolism-improving drugs]

Alfacalcidol, calcitriol, menatetrenone, elcatonin, salmon calcitonin, alendronate sodium hydrate, disodium etidronate, disodium pamidronate, disodium incadronate, ipriflavone, etc.

### [Amino acids]

L-asparatic acid and salts thereof, aminoethylsulfonic acid, etc.

### [Vitamin preparations]

Retinol palmitate, alfacalcidol, thiamin hydrochloride, riboflavin, panthenol, cyanocobalamin, pyridoxine hydrochloride, nicotinic acid, folic acid, calcium pantothenate, ascorbic acid, tocopherol acetate, tocopherol nicotinate, biotin, phytonadione, vitamin complexes, etc.

### [Hemostatic agents]

Carbazochrome sodium sulfonate, adrenochrome guanylhydrazone mesylate, ε-aminocaproic acid, tranexamic acid, polidocanol, monoethanolamine oleate, hemocoagulase, thrombin, etc.

### [Antithrombotic agents]

Conjugated estrogen, urokinase, tisokinase, alteplase, nasaruplase, nateplase, monteplase, pamiteplase, batroxobin, ticlopidine hydrochloride, cilostazol, limaprost alfadex, sodium ozagrel, argatroban, ethyl icosapentate, sarpogrelate hydrochloride, heparin calcium, heparin sodium, protamine sulfate, dalteparin sodium, warfarin, warfarin calcium, sodium citrate, Antithrombin III (gene recombinant), epoetin alfa (gene recombinant), epoetin beta (gene recombinant), lenograstim, filgrastim, nartograstim, etc.

### [Antibiotics]

Penicillin G, penicillin V, benzylpenicillin calcium, benzylpenicillin benzathine, phenethicillin potassium, cloxacillin sodium, flucloxacillin sodium, ampicillin, amoxicillin, hetacillin, ciclacillin, carbenicillin, talampicillin hydrochloride, ticarcillin sodium, sulbenicillin, sulbenicillin sodium, carindacillin sodium, piperacillin sodium, pivmecillinam hydrochloride, sultamicillin tosilate, clavulanate potassium, phenoxymethyl penicillin potassium, phenoxymethyl penicillin, dicloxacillin sodium, oxacillin, cloxacillin, clavulanate potassium or ticarcillin sodium, sulbactam sodium, ampicillin, ampicillin sodium, cefaloridine, cefazolin, cephaloglycin, benzathine, cephalothin sodium, cefalexin, cefamandole sodium, cefuroxime sodium, cefotaxime sodium, ceftizoxime sodium, ceftazidime, cefbuperazone sodium, latamoxef sodium, flomoxef sodium, aztreonam, carumonam sodium, meropenem, imipenem, cilastatin sodium, panipenem, betamipron, gentamicin sulfate, kanamycin sulfate, tobramycin, streptomycin, dibekacin, fradiomycin, fosfomycin, vancomycin hydrochloride, tetracycline hydrochloride, oxytetracycline, dimethyl chlorotetracycline, doxycycline, minocycline, erythromycin, leucomycin, josamycin, kitasamycin, roxithromycin, lincomycin hydrochloride, clindamycin, amikacin, gramicidin, gramicidin S, capreomycin, cycloserine, enviomycin, rifampicin, nystatin, trichomycin, phosphonomycin, amphotericin B, chloramphenicol, thiamphenicol, griseofulvin, variotin, pyrrolnitrin, siccanin, nitrofurantoin, cefamezin, etc.

### [Sulfa drugs]

Mafenide acetate, sulfadiazine, sulfadiazine silver, sulfamethoxazole sodium, sulfadimethoxine, sulfamethoxazole, sulfamonomethoxine, sulfisomidine, sulfisomidine sodium, etc.

### [Antimycotic agents]

Naftiomate, clotrimazole, cloconazole hydrochloride, isoconazole nitrate, sulconazole nitrate, fluconazole, itraconazole, miconazole, ketoconazole, thioconazole, bifonazole, griseofulvin, siccanin, trichomycin, pimaricin, amphotericin B, nystatin, pyrrolnitrin, exalamide, tolciclate, variotin, haloprogin, phenyliodo undecynoate, flucytosine, terbinafine hydrochloride, naftifine, octpirox, ciclopirox, olamine, acyclovir, idoxuridine, etc.

### [Antiviral agents]

Acyclovir, valacyclovir hydrochloride, vidarabine, ganciclovir, amantadine, vidarabine, inosine pranobex, amantadine hydrochloride, oseltamivir phosphate, ribavirin, etc.

### [Anti-HIV agents]

Zidovudine, azidothymidine, sanilvudine, zalcitabine, didanosine, lamivudine, abacavir sulfate, efavirenz, nevirapine, delavirdine mesylate, saquinavir, saquinavir mesylate, nelfinavir mesylate, ritonavir, indinavir sulfate, amprenavir, etc.

### [Antiparasitic and antiprotozoal agents]

Quinine, metronidazole, tinidazole, sulfadoxine, pyrimethamine, mefloquine hydrochloride, pyrantel pamoate, mebendazole, tiabendazole, diethylcarbamazine citrate, praziquantel, pentamidine isetionate, etc.

### [Antirheumatic drugs]

Bucillamine, salazosulfapyridine, penicillamine, actarit, lobenzarit, sodium aurothiomalate, auranofin, D-penicillamine, prednisolone farnesylate, etc.

### [Immunosuppressants]

Ciclosporin, azathioprine, mizoribine, muromonab-CD3, tacrolimus hydrate, gusperimus hydrochloride, mycophenolate mofetil, methotrexate, cyclophosphamide, etc.

### [Sunscreen agents]

p-Amino benzoate, p-dimethyl aminobenzoate, etc.

### [Antiallergic agents]

Cyproheptadine hydrochloride, tranilast, sodium cromoglicate, amlexanox, repirinast, tazanolast, pemirolast potassium, ketotifen fumarate, oxatomide, mequitazine, fexofenadine hydrochloride, ebastine, cetirizine hydrochloride, zafirlukast, montelukast sodium, emedastine fumarate, etc.

### [Ophthalmic preparations]

Chondroitin sodium sulfate, neostigmine methyl sulfate, glutathione, distigmine bromide, sodium cromoglicate, etc.

### [Antipsoriatic agents]

Methoxsalen, etc.

### [Skin softeners and emollients]

Hydroquinone, heparin, urea, chondroitin sulfate, etc.

### [Moisturizing agents]

Ceramide, hyaluronic acid, coenzyme Q10, isoflavone, LIPIDURE^{®}, sericin, etc.

### [Baldness-remedies and hair growth stimulants]

Carpronium hydrochloride, minoxidil, cepharanthine, swertia herb extract, vitamin E derivatives, ginseng extract, nicotinamide, benzyl nicotinate, Sophora root extract, *Panacis japonici rhizoma,* biotin, pentadecanoate glyceride, acetyl methionine, inositol, L-serine, pyridoxine hydrochloride, calcium pantothenate, estradiol benzoate, ethynylestradiol, diethyl stilbestrol, testosterone, methyltestosterone, hydrocortisone, diphenhydramine, *Polygoni radix,* takanal, etc.

### [Peptide hormone preparations]

Insulin, angiotensin, vasopressin, felypressin, protirelin, gonadotropin-releasing hormone, corticotropin, prolactin, somatropin, thyrotropin, luteinizing hormone, calcitonin, kallikrein, parathyrin, glucagon, oxytocin, gastrin, secretin, serum gonadotropin, etc.

### [Anti-smoking aids]

Nicotine, bupropion hydrochloride, etc.

### [Diet ingredients]

Garcinia, capsaicin, citrus, Gymnema, guava, melilot, green papaya extract, niacin, psyllium, etc.

### [Skin whitening ingredients]

Hydroquinone, alpha arbutin, vitamin C, kojic acid, gallogen, camomile extract, Rucinol, etc.

### [Anti-aging ingredients]

Coenzyme Q10, melatonin, DHEA, tretinoin, ajoene, etc.

### [Polysaccharides]

Heparin, chondroitin sodium sulfate, etc.

### [Plant extracts and essential oils]

*Rubia cordifolia* root, *Vitis vinifera,* Agaricus, *Akebia quinata,* thistle, *Angelica keiskei,* gambir, avocado, althea, arnica, aloe vera, *Epimedium grandiflorum, Polygonatum officinale,* ginkgo leaves, Ylang Ylang, fennel, curcuma, cuttlebone, *Mume fructus, Linderae radix,* Limonite, *Acanthopanax senticosus Harms,* Corydalis Tuber, Astragalus root, Scutellaria root, Phellodendron Bark, coptis root, plantain, olive, orange, *Hypericum erectum, Digenea simplex, Erythrina indica,* prunellae spica, white oak, *Polygoni radix, Stachys betonica,* pueraria root, Talcum, chamolile, *Torreya nucifera,* trichosanthes seed, licorice, *Eclipta prostrata,* Platycodon root, *Chrysanthemum morifolium,* orange pease, cinchona, catnip, apricot kernel, cucumber, *Polygonatum officinalle,* Cherokee Rose, *Lysimachia christinae,* Sophora root, grapefruit, schizonepeta herb, cassia buds, cassia bark, seed of *Euryale ferox Salisb.,* cassia seed, gentian, geranium herb, Cyperus Rhizome, Magnolia Bark, oriental bezoar, *Acanthopanax Senticosus Harms ,* coconut, pepper, Arctii Fructus, amber, wheat germ, comfrey, Asiasarum root, cypress, saffron, hawthorn, Zanthoxyli Fructus, rehmanniae radix, *Luffa cylindrica,* Lithosermi Radix, perilla, *Perillae frutescens,* citronella, lime, cinnamon, *Filipendula multijuga,* Halloysitum rubrum, peony, *Betula alba, Equisetum arvense, Salvia officinalis, Citrus reticulata,* red *Paeonia lactiflora,* sesame, *Haliotis diversicolor,* mallow, Geranium, *Agrimonia pilosa,* Cnidii Rhizoma, St. John's wort, Mulberry bark, Turmeric, Rhubarb, soy bean extract, fermented soy bean extract, Zizyphi Fructus, thyme, tea extract, clove, Citrus Unshiu Peel, tea tree, *Indosasa crassiflora* McClure, Sweet Tea *(Rubus suavissimus), Panax notoginseng,* wax gourd, Japanese angelica root, *Calendula officinalis, Cordyceps sinensis,* peach kernel, bitter orange peel, *Houttuynia cordata, Cuscuta chinensis* seed, *Eucommia ulmoides, Potentilla anserina,* ginseng, garlic, *Ligustrum japonicum,* neroli, malt, *Dictamnus dasycarpus, Pulsatilla chinensis,* Ophiopogon tuber, basil, parsley, Coix lacryma-joli, vanilla, Glehnia root, hamamelis, rose, heath, Pycnogenol, *Trapa japonica,* hinokitiol, sandalwood, Eriobotrya, betel nut, hoelen, aconiti tuber, blueberry, betaine, safflower, peppermint, henna, *Impatiens balsamina* extract, saposhnikovia root, peony, hop, jojoba, *Citrus reticulata* fruit, ephedra herba, pine needle, morronnier, mandarine, mint, *Fomes yucatensis, Melissa officinalis, Equisetum hiemale,* Saussurea root, peach leaf, centaury, cornflower, eucalyptus, Saxifraga, *Citrus junos,* coix seed, mugwort, *Momordica grosvenori,* lavender, Rooibos, lemon, lemongrass, lemon verbena, forsythia, rosewood, rose hip, rosemary, etc.

### [Enzymes]

Trypsin, papain, protease, serrapeptase, lysozyme, bromelain, streptokinase, plasmin, urokinase, α-chymotrypsin, serratio peptidase, semi-alkaline peptidase, lysozyme chloride, etc.

### [Vaccines]

Yellow fever, measles, rubella, chickenpox, tuberculosis, polio, cholera, typhoid, rabies, Japanese encephalitis, hepatitis, diphtheria, pertussis, tetanus, influenza, pest, tick-borne encephalitis, meningitis, etc.

The dosage forms used in the present invention are not particularly limited. Any conventional dosage forms used in contact with the skin or mucosa can be used, including, for example, a cream, ointment, liquid, liniment, lotion, emulsion, powder, foam, tape, patch, cataplasm, plaster, suppository, nasal drop, eye drop, vaginal preparation, dentifrice, and chewing gum.

Herein below, the present invention will be described using Examples, however, it is not to be construed as being limited thereto.

### Best Mode for Carrying Out the Invention

The present invention is illustrated in detail below using Examples.

### [Example 1: Comparative study of hydroxyapatites (hereinafter abbreviated as "HAP") with different particle sizes on skin permeability]

Experiments on skin permeability of HAPs with different particle sizes were carried out using the method of Fujii (2) with modification.

Eight-week old female hairless rats were purchased from Saitama Experimental Animal Stock Center and used in this experiment. Rat abdominal skin was excised, and after fat was carefully removed, the skin was stored at -80°C until use. The frozen skin was thawed at room temperature for about 30 minutes. The resulting full-thickness skin was used in the permeability study.

An improved Franz cell developed by Fujii was used in the skin permeability study. The cell was mounted so as to have the skin inserted between a lower chamber (receptor phase: dermis side) and an upper chamber (donor phase: horny layer side). Samples are to be added to the upper chamber. 16 ml of isotonic phosphate buffer (pH 7.1) was incubated at 37°C in the receptor phase while being stirred with a stirrer. 0.5 ml of each sample was added to the donor phase. During the experiment, the top of the cell was sealed with laboratory film.

Samples added to the donor phase were: non-treated control group - isotonic phosphate buffer containing no additives (Controlled study 1); suspension of 1 weight percent HAP with a maximum particle size of 0.05 µm or less (mean particle size was about 0.03 µm) in isotonic phosphate buffer (Study 1-1); suspension of 1 weight percent HAP with a maximum particle size of 0.1 µm or less (mean particle size was about 0.06 µm) in isotonic phosphate buffer (Study 1-2); suspension of 1 weight percent HAP with a maximum particle size of 1 µm or less (mean particle size was about 0.5 µm) in isotonic phosphate buffer (Study 1-3); and suspension of 1 weight percent HAP with a maximum particle size of 20 µm or less (mean particle size was about 8 µm) in isotonic phosphate buffer (Study 1-4). The study was conducted using the samples described above.

In this experiment, calcium concentration in the receptor phase for each sample was measured and calculated after 24 hours; the results are shown in Table 1 as the amount of HAP permeated through the skin. ICP was used for ion measurement.

**Table 1**

| | Skin permeability of HAP |
|---|---|
| Study 1-1 | 98% |
| Study 1-2 | 94% |
| Study 1-3 | 75% |
| Study 1-4 | 32% |
| Controlled study 1 | 0.7% |

As seen in Table 1, in comparison with HAPs with larger particle sizes, the skin permeability is greater for HAPs with a maximum particle size of 1.0 µm or less, and even greater for HAPs with 0.1 µm or less maximum particle size. In Controlled study 1 without HAP, a small amount of calcium was detected. Although this value was recorded as an HAP amount in Table 1, it accounts for the endogenous calcium in the skin.

### [Example 2: Comparative study of HAPs with different particle sizes on mucosa permeability]

Eight-week old female golden hamsters were used in this experiment. The animals were sacrificed by decapitation to avoid influences of anesthesia. Immediately, oral mucosa was excised. The oral mucosa was then excised so as to include mucosa to basal membrane with considerable care not to let it dry.

An improved Franz cell was used in the mucosa permeability study. The cell comprised an upper chamber (donor phase) to which a sample is added, and a lower chamber (receptor phase); and the oral mucosa was placed in between. 16 ml of isotonic phosphate buffer (pH 7.1) containing kanamycin was incubated at 37°C in the receptor phase while being stirred with a stirrer. 0.5 ml of each sample was added to the donor phase. During the experiment, the top of the cell was sealed with laboratory film.

Samples added to the donor phase were: non-treated control group - isotonic phosphate buffer containing no additives (Controlled study 2); suspension of 1 weight percent HAP with a maximum particle size of 0.05 µm or less (mean particle size was about 0.03 µm) in isotonic phosphate buffer (Study 2-1); suspension of 1 weight percent HAP with a maximum particle size of 0.1 µm or less (mean particle size was about 0.06 µm) in isotonic phosphate buffer (Study 2-2); suspension of 1 weight percent HAP with a maximum particle size of 1 µm or less (mean particle size was about 0.5 µm) in isotonic phosphate buffer (Study 2-3); and suspension of 1 weight percent HAP with a maximum particle size of 20 µm or less (mean particle size was about 8 µm) in isotonic phosphate buffer (Study 2-4).

In this experiment, calcium concentration in the receptor phase for each sample was measured and calculated after 24 hours; the results are shown in Table 2 as the amount of HAP permeated through the mucosa. ICP was used for ion measurement.

**Table 2**

| | Mucosa permeability of HAP |
|---|---|
| Study 2-1 | 95% |
| Study 2-2 | 89% |
| Study 2-3 | 68% |
| Study 2-4 | 22% |
| Controlled study 2 | 0.9% |

As seen in Table 2, in comparison with HAPs with larger particle sizes, the mucosa permeability is greater for HAPs with a maximum particle size of 1.0 µm or less, and even greater for HAPs with a maximum particle size of 0.1 µm or less.

### [Preparation of compositions for drug permeability study]

Table 3 shows compositions used in the comparative studies of drug permeability using HAPs with different particle sizes. Ethanol was used in the preparation of test compositions comprising indomethacin which is insoluble in water. A composition that does not contain HAP was prepared as a control (Comparative example 1).

**Table 3**

| | Example 3 | Example 4 | Example 5 | Comparative example 1 |
|---|---|---|---|---|
| Indomethacin | 1% | 1% | 1% | 1% |
| Ethanol | 40% | 40% | 40% | 40% |
| Hydroxyapatite (maximum particle size: 0.1 µm or less) | 1% | - | - | - |
| Hydroxyapatite (maximum particle size: 1 µm or less) | - | 1% | - | - |
| Hydroxyapatite (maximum particle size: 20 µm or less) | - | - | 1% | - |
| Purified water | remaining amount | remaining amount | remaining amount | remaining amount |
| Total | 100% | 100% | 100% | 100% |

The compositions (ointment) used in the comparative studies of drug permeability for different HAP contents are shown in Tables 4 and 5. In Example 9, indomethacin and HAP were first mixed using a stirrer, and then combined with the remaining agents. In Example 10, all ingredients are mixed simultaneously and used. For the controls, a composition comprising 1500 weight percent HAP based on the drug (Comparative example 2) and a composition that does not contain HAP (Comparative example 3) were prepared.

**Table 4**

| | Example 6 | Example 7 | Example 8 | Example 9 | Example 10 |
|---|---|---|---|---|---|
| Indomethacin | 1% | 1% | 1% | 1% | 1% |
| White vaseline | 25% | 25% | 25% | 25% | 25% |
| Stearyl alcohol | 22% | 22% | 22% | 22% | 22% |
| Propylene glycol | 12% | 12% | 12% | 12% | 12% |
| Sodium lauryl sulfate | 1% | 1% | 1% | 1% | 1% |
| Hydroxyapatite (maximum particle size: 0.1 µm or less) | 0.001% | 0.01% | 0.1% | 1% | 1% |
| Purified water | remaining amount | remaining amount | remaining amount | remaining amount | remaining amount |
| Total | 100% | 100% | 100% | 100% | 100% |

**Table 5**

| | Example 11 | Example 12 | Example 13 | Comparative example 2 | Comparative example 3 |
|---|---|---|---|---|---|
| Indomethacin | 1% | 1% | 1% | 1% | 1% |
| White vaseline | 25% | 25% | 25% | 25% | 25% |
| Stearyl alcohol | 22% | 22% | 22% | 22% | 22% |
| Propylene glycol | 12% | 12% | 12% | 12% | 12% |
| Sodium lauryl sulfate | 1% | 1% | 1% | 1% | 1% |
| Hydroxyapatite (maximum particle size: 0.1 µm or less) | 2% | 5% | 10% | 15% | - |
| Purified water | remaining amount | remaining amount | remaining amount | remaining amount | remaining amount |
| Total | 100% | 100% | 100% | 100% | 100% |

### [Study 3: Comparative study of the skin permeability of indomethacin for HAPs with different particle sizes]

The experiment was conducted using the abdominal skin of hairless rats.

16 ml of isotonic phosphate buffer (pH 7.1) was incubated at 37°C in the receptor phase (dermis side) while being stirred with a stirrer. The samples (1 g each) in Examples 3 to 5 and Comparative example 1 were added (applied area, 3.14 cm²) to the donor phase (horny layer side). The top was sealed with laboratory film (Studies 3-1 to 3-3, and Controlled study 3).

The solutions in the receptor phase were sampled at 1, 3, 6, 12, and 24 hours after the start of study. The concentrations of drugs in the solutions were quantified by high performance liquid chromatography to determine the amounts of drugs permeated through the skin. The result is shown in Table 6; the values represent skin permeability in µg/cm².

**Table 6**

| Time | Study 3-1 (Example 3) | Study 3-2 (Example 4) | Study 3-3 (Example 5) | Controlled study 3 (Comparative example 1) |
|---|---|---|---|---|
| 1 | 44.7 | 7.9 | 4.6 | 2.3 |
| 3 | 140.0 | 23.8 | 8.2 | 6.4 |
| 6 | 250.6 | 56.1 | 19.5 | 10.0 |
| 12 | 391.2 | 139.4 | 47.2 | 16.1 |
| 24 | 545.8 | 370.1 | 153.3 | 62.5 |

### [Study 4: Comparative study of the mucosa permeability of indomethacin for HAPs with different particle sizes]

The experiment was conducted using oral mucosa from golden hamsters.

16 ml of isotonic phosphate buffer (pH 7.1) was incubated at 37°C in the receptor phase (basal side) while being stirred with a stirrer. The samples (1 g each) in Examples 3 to 5 and Comparative example 1 were added (applied area, 3.14 cm²) to the donor phase (mucosal side). The top was sealed with laboratory film (Studies 4-1 to 4-3, and Controlled study 4).

The solutions were sampled from the receptor phase at 1, 3, 6, 12, and 24 hours after the start of study. The concentrations of drugs in the solutions were quantified by high performance liquid chromatography to determine the amounts of drugs permeated through the skin. The result is shown in Table 7; the values represent mucosa permeability in µg/cm².

**Table 7**

| Time | Study 4-1 (Example 3) | Study 4-2 (Example 4) | Study 4-3 (Example 5) | Controlled study 4 (Comparative example 1) |
|---|---|---|---|---|
| 1 | 30.6 | 5.4 | 2.8 | 1.2 |
| 3 | 95.5 | 19.9 | 4.3 | 2.5 |
| 6 | 186.3 | 48.7 | 11.9 | 6.3 |
| 12 | 332.8 | 127.2 | 33.0 | 11.1 |
| 24 | 512.7 | 311.6 | 98.1 | 37.4 |

### [Study 5: Comparative study of the skin permeability of indomethacin with different HAP contents]

The experiment was conducted using the abdominal skin of hairless rats.

16 ml of isotonic phosphate buffer (pH 7.1) was incubated at 37°C in the receptor phase (dermis side) while being stirred with a stirrer. The samples (1 g each) in Examples 6 to 13 and Comparative examples 2 and 3 were added (applied area, 3.14 cm²) to the donor phase (horny layer side). The top was sealed with laboratory film (Studies 5-1 to 5-8 and Controlled studies 5-1 to 5-2).

The solutions were sampled from the receptor phase 24 hours after the start of study. The concentrations of drugs in the solutions were quantified by high performance liquid chromatography to determine the amounts of drugs permeated through the skin. The result is shown in Table 8; the values represent skin permeability (µg/cm²).

**Table 8**

| | Skin permeability (µg/cm²) |
|---|---|
| Study 5-1 | 189.2 |
| Study 5-2 | 237.4 |
| Study 5-3 | 275.7 |
| Study 5-4 | 315.8 |
| Study 5-5 | 280.1 |
| Study 5-6 | 284.3 |
| Study 5-7 | 279.0 |
| Study 5-8 | 270.5 |
| Controlled study 5-1 | 251.4 |
| Controlled study 5-2 | 21.9 |

When compared with the Controlled study 5-2 composition containing no HAP, the skin permeability of the Study 5-1 composition which has a HAP content of 0.1 weight percent based on the drug was markedly enhanced; and the skin permeability was significantly enhanced in the compositions of Studies 5-3 to 5-6, in which the HAP content based on the drug ranged from 10 to 200 weight percent (see Tables 4 and 5). The skin permeability was particularly high in the Study 5-4 composition, which was prepared by pre-mixing the drug and HAP with a stirrer and then combining the mixture with the remaining agents.

### [Example 14: Skin permeability study of various drugs]

Table 9 shows compositions used in the skin permeability study of various drugs (Examples 14-1 to 14-16). Compositions that do not contain HAP were prepared as controls (Comparative examples 4-1 to 4-16).

**Table 9**

| | Ingredient | Content (%) |
|---|---|---|
| Example 14-1 | naloxone hydrochloride | 1.0 |
| | HAP | 0.1 |
| | glycerin | 10.0 |
| | purified water | remainder |
| Example 14-2 | insulin | 1.0 |
| | HAP | 0.01 |
| | dextrin | 5.0 |
| | purified water | remainder |
| Example 14-3 | azidothymidine | 1.0 |
| | HAP | 1.0 |
| | ethanol | 10.0 |
| | purified water | remainder |
| Example 14-4 | eperisone hydrochloride | 1.0 |
| | HAP | 0.1 |
| | purified water | remainder |
| Example 14-5 | ephedrine hydrochloride | 1.0 |
| | HAP | 0.01 |
| | purified water | remainder |
| Example 14-6 | fluphenazine decanoate | 1.0 |
| | HAP | 2.0 |
| | ethanol | 5.0 |
| | purified water | remainder |
| Example 14-7 | colchicine | 1.0 |
| | HAP | 0.1 |
| | ethanol | 2.0 |
| | purified water | remainder |
| Example 14-8 | sodium valproate | 1.0 |
| | HAP | 3.0 |
| | purified water | remainder |
| Example 14-9 | atropine sulfate | 1.0 |
| | HAP | 1.0 |
| | purified water | remainder |
| Example 14-10 | meclofenoxate hydrochloride | 1.0 |
| | HAP | 0.01 |
| | purified water | remainder |
| Example 14-11 | trimetazidine hydrochloride | 1.0 |
| | HAP | 0.1 |
| | purified water | remainder |
| Example 14-12 | todralazine hydrochloride | 1.0 |
| | HAP | 1.0 |
| | purified water | remainder |
| Example 14-13 | clofibrate | 1.0 |
| | HAP | 5.0 |
| | ethanol | 1.0 |
| | purified water | remainder |
| Example 14-14 | dinoprost | 1.0 |
| | HAP | 0.5 |
| | ethanol | 2.0 |
| | purified water | remainder |
| Example 14-15 | nicotine | 1.0 |
| | HAP | 3.0 |
| | purified water | remainder |
| Example 14-16 | niacin | 1.0 |
| | HAP | 0.1 |
| | purified water | remainder |

### [Study 6: Comparative study of skin permeability for various drugs]

The skin permeability study was conducted using the abdominal skin of hairless rats.

16 ml of isotonic phosphate buffer (pH 7.1) was incubated at 37°C in the receptor phase (dermis side) while being stirred with a stirrer. The samples (1 g each) in Examples 14-1 to 14-16 and Comparative examples 4-1 to 4-16 were added (applied area, 3.14 cm²) to the donor phase (horny layer side). The top was sealed with laboratory film (Studies 6-1 to 6-16, and Controlled studies 6-1 to 6-16).

The solutions were sampled from the receptor phase 24 hours after the start of study. The concentrations of drugs in the solutions were quantified by high performance liquid chromatography to determine the amounts of drugs permeated through the skin. The result is shown in Table 10; the values represent skin permeability (µg/cm²).

**Table 10**

| | Skin permeability (µg/cm²) | | Skin permeability (µg/cm²) |
|---|---|---|---|
| Study 6-1 | 373.9 | Controlled study 6-1 | 35.1 |
| Study 6-2 | 43.7 | Controlled study 6-2 | 7.3 |
| Study 6-3 | 614.1 | Controlled study 6-3 | 41.8 |
| Study 6-4 | 405.6 | Controlled study 6-4 | 32.0 |
| Study 6-5 | 541.0 | Controlled study 6-5 | 47.2 |
| Study 6-6 | 136.5 | Controlled study 6-6 | 14.6 |
| Study 6-7 | 250.7 | Controlled study 6-7 | 20.2 |
| Study 6-8 | 898.3 | Controlled study 6-8 | 64.7 |
| Study 6-9 | 82.4 | Controlled study 6-9 | 11.4 |
| Study 6-10 | 359.2 | Controlled study 6-10 | 30.5 |
| Study 6-11 | 309.0 | Controlled study 6-11 | 25.6 |
| Study 6-12 | 671.8 | Controlled study 6-12 | 43.4 |
| Study 6-13 | 634.9 | Controlled study 6-13 | 45.9 |
| Study 6-14 | 310.6 | Controlled study 6-14 | 24.8 |
| Study 6-15 | 1057.3 | Controlled study 6-15 | 66.0 |
| Study 6-16 | 1296.1 | Controlled study 6-16 | 69.3 |

Naloxone hydrochloride, a narcotic antagonist, used in Example 14-1 has a structure similar to those of morphine and codeine, which are narcotic analgesic agents. Therefore, these narcotic analgesic agents may also give a result similar to thoseobtained in Study 6-1 and Controlled study 6-1.

| [Example 15-1: Cream] | |
|---|---|
| Tolbutamide | 10.0 |
| HAP (muximum particle size is 0.1 µm or less) | 1.0 |
| Cetanol | 5.0 |
| Polyethylene glycol | 3.0 |
| Glycerin | 5.0 |
| Liquid paraffin | 10.0 |
| HAP (muximum particle size is 0.1 µm or less) | 1.0 |
| Purified water | remainder |
| Total | 100.0 |

| [Example 15-2: Liquid preparation] | |
|---|---|
| Minoxidil | 0.5 |
| Dipotassium glycyrrhizinate | 0.1 |
| Pantothenyl alcohol | 0.2 |
| Cinchona bark extract | 1.0 |
| HAP (maximum particle size is 0.1 µm or less) | 1.0 |
| Polyoxyethylene hydrogenated castor oil | 0.3 |
| Propylene glycol | 5.0 |
| HAP (maximum particle size is 0.1 µm or less) | 1.0 |
| Ethanol | 60.0 |
| Purified water | remainder |
| Total | 100.0 |

| [Example 15-3: Tape] | |
|---|---|
| Nicotine | 15.0 |
| HAP (maximum particle size is 0.1 µm or less) | 15.0 |
| Ester gum | 12.0 |
| Silicone adhesive | 58.0 |
| Total | 100.0 |

| [Example 15-4: Patch] | |
|---|---|
| Nitroglycerin | 14.0 |
| HAP (maximum particle size is 0.1 µm or less) | 5.0 |
| Ester gum | 13.0 |
| Silicone adhesive | 68.0 |
| Total | 100.0 |

| [Example 15-5: Cataplasm] | |
|---|---|
| Somatropin | 0.5 |
| HAP (maximum particle size is 0.1 µm or less) | 5.0 |
| L-Histidine | 0.1 |
| Sodium polyacrylate | 2.0 |
| Gelatin | 5.0 |
| Polyvinyl alcohol | 1.0 |
| Glycerin | 15.0 |
| Sorbitol | 15.0 |
| Polyoxyethylene glycol | 1.0 |
| Kaolin | 2.0 |
| Monolaurate polyethylene glycol | 1.5 |
| Castor oil | 1.0 |
| 1,3-Butylene glycol | 3.0 |
| Salicylate glycol | 1.0 |
| Purified water | remainder |
| Total | 100.0 |

| [Example 15-6: Suppository or vaginal preparation] | |
|---|---|
| Diazepam | 1.0 |
| HAP (maximum particle size is 0.1 µm or less) | 0.01 |
| Polyoxyethylene polyoxypropylene glycol | remainder |
| Total | 100.0 |

| [Example 15-7: Nasal drop] | |
|---|---|
| Dipotassium glycyrrhizinate | 0.2 |
| Chlorpheniramine maleate | 0.1 |
| HAP (maximum particle size is 0.1 µm or less) | 0.05 |
| Potassium hydrogen phosphate | 0.01 |
| Benzalkonium chloride | 0.01 |
| Propylene glycol | 0.5 |
| Purified water | remainder |
| Total | 100.0 |

| [Example 15-8: Eye drop] | |
|---|---|
| Neostigmine methyl sulfate | 0.005 |
| d- α-Tocopherol acetate | 0.02 |
| Sodium chondroitin sulfate | 0.1 |
| Neostigmine methyl sulfate | 0.005 |
| HAP (maximum particle size is 0.1 µm or less) | 0.0002 |
| Sodium edentate | 0.1 |
| Propylene glycol | 0.5 |
| Potassium sorbate | 0.1 |
| Sodium chloride | 0.3 |
| Purified water to | remainder |
| Total | 100.0 |

| [Example 15-9: Dentifrice] | |
|---|---|
| Garcinia extract | 20.0 |
| HAP (maximum particle size is 0.1 µm or less) | 0.02 |
| Glycerin | 20.0 |
| Polyethylene glycol | 3.0 |
| Sodium lauryl sulfate | 1.0 |
| Xanthan gum | 0.5 |
| Hydroxyethyl cellulose | 0.5 |
| Menthol | 0.5 |
| Purified water | remainder |
| Total | 100.0 |

| [Example 15-10: Chewing gum] | |
|---|---|
| Doxifluridine | 5.0 |
| HAP (maximum particle size is 0.1 µm or less) | 0.5 |
| Gum base | 28.0 |
| Xylitol | 30.0 |
| Palatinite | 21.0 |
| Maltitol | 3.8 |
| Softener | 0.8 |
| Flavor | 1.0 |
| Maltitol syrup | remainder |
| Total | 100.0 |

### [Study 7: Preparation of various dosage forms of transdermal and transmucosal compositions, and skin and mucosa permeability studies]

Compositions were prepared in various dosage forms. In Examples 15-1 to 15-5, the compositions were studied for their skin permeability by the same method described in Example 14 (Studies 7-1 to 7-5). In Examples 15-6 to 15-10, the compositions were tested for their mucosa permeability as in Example 4. The chewing gum composition described in Example 15-10 was tested without a gum base (Studies 7-6 to 7-10). Compositions that do not contain HAP were prepared and tested as controls in Examples 15-1 to 15-10 (Controlled studies 7-1 to 7-10).

These results are shown in Table 11.

**Table 11**

| | Skin permeability (µg/cm²) | | Skin permeability (µg/cm²) |
|---|---|---|---|
| Study 7-1 | 1596.1 | Controlled study 7-1 | 127.4 |
| Study 7-2 | 285.2 | Controlled study 7-2 | 20.6 |
| Study 7-3 | 91.5 | Controlled study 7-3 | 15.9 |
| Study 7-4 | 84.7 | Controlled study 7-4 | 17.3 |
| Study 7-5 | 7.4 | Controlled study 7-5 | 2.1 |

| | Mucosa permeability (µg/cm²) | | Mucosa permeability (µg/cm²) |
|---|---|---|---|
| Study 7-6 | 152.9 | Controlled study 7-6 | 17.5 |
| Study 7-7 | 38.3 | Controlled study 7-7 | 9.2 |
| Study 7-8 | 4.6 | Controlled study 7-8 | 0.8 |
| Study 7-9 | 23.8 | Controlled study 7-9 | 7.0 |
| Study 7-10 | 421.4 | Controlled study 7-10 | 43.7 |

The results described above show that the transdermal absorption and the transmucosal absorption of various ingredients are enhanced through combination of hydroxyapatite.

### Industrial Applicability

The present invention provides transdermal and transmucosal preparations that enhance the transdermal/transmucosal absorption of essential ingredients when applied to the skin or mucosa.

## Claims

1. A transdermal and/or transmucosal preparation comprising an ingredient to be absorbed transdermally and/or transmucosally and a hydroxyapatite.

2. The transdermal and/or transmucosal preparation of claim 1, wherein the hydroxyapatite has a maximum particle size of 1 µm or less.

3. The transdermal and/or transmucosal preparation of claim 1, wherein the hydroxyapatite has a maximum particle size of 0.1 µm or less.

4. The transdermal and/or transmucosal preparation of any one of claims 1 to 3, wherein the hydroxyapatite has a content of 0.1 % to 1000% based on a drug to be combined.

5. The transdermal and/or transmucosal preparation of any one of claims 1 to 4, wherein the ingredient to be absorbed transdermally and/or transmucosally comprises one or more selected from the group consisting of: an analgesic, antipyretic, and anti-inflammatory agent, antigout or antihyperuricemic agent, nonsteroidal anti-inflammatory agent, steroidal anti-inflammatory agent, wound healing agent, anticancer agent, hypnotic or analgesic agent, anxiolytic agent, antipsychotic agent, antidepressant agent, antimanic agent, antihistaminic agent, antiepileptic agent, local anesthetic, therapeutic agent for peripheral circulation disorders, antiparkinson agent, muscle relaxant, autonomic or antispasmodic agent, antidiaphoretic agent, cerebral circulation or metabolism-improving drug, cardiac stimulant, antianginal agent, β blocker, Ca antagonist, antiarrhythmic agent, antihypertensive agent, vasodilator, decongestant, capillary stabilizer, antihyperlipemic agent, vasopressor, respiratory stimulant, bronchodilator or antiasthmatic agent, antitussive agent, expectorant, therapeutic agent for peptic ulcer, deglutitory agent, antiemetic agent, obstipant or antiflatulent, stomachic or digestant, cathartic, hemorrhoidal preparation, therapeutic for neurogenic bladder, therapeutic for benign prostatic hyperplasia or frequent urination, therapeutic for nephrolithiasis or urolithiasis, diuretic agent, choleretic agent, antidiabetic agent, pituitary hormone preparation, adrenocortical hormone preparation, sex hormone preparation, prostaglandin, therapeutic agent for thyroid dysfunction, anti-osteoporosis or bone metabolism improving drug, amino acids vitamin preparation, hemostatic agent, antithrombotic agent, antibiotic, sulfa drug, antimycotic agent, antiviral agents, anti-HIV agents, antiparasitic or antiprotozoal agents, antirheumatic drugs, immunosuppressant, sunscreen agent, antiallergic agent, ophthalmic preparation, antipsoriatic agent, skin softener or emollient, moisturizing ingredient, baldness-remedy or hair growth stimulant, peptide hormone preparation, anti-smoking aid, diet ingredient, skin whitening ingredient, anti-aging ingredient, polysaccharide, plant extract or essential oil, enzyme, and vaccine.

6. The transdermal and/or transmucosal preparation of any one of claims 1 to 5, wherein a dosage form of the preparation is a cream, ointment, liquid, tape, patch, cataplasm, suppository, nasal drop, eye drop, vaginal preparation, dentifrice, or chewing gum.

## Amended claims

### Amended claims under Art. 19.1 PCT

**1.** A transdermal preparation comprising an ingredient to be absorbed transdermally and a hydroxyapatite.

**2.** The transdermal preparation of claim 1, wherein the hydroxyapatite has a maximum particle size of 1 µm or less.

**3.** The transdermal preparation of claim 1, wherein the hydroxyapatite has a maximum particle size of 0.1 µm or less.

**4.** The transdermal preparation of any one of claims 1 to 3, wherein the hydroxyapatite has a content of 0.1 % to 1000% based on a drug to be combined.

**5.** The transdermal preparation of any one of claims 1 to 4, wherein the ingredient to be absorbed transdermally comprises one or more selected from the group consisting of: an analgesic, antipyretic, and anti-inflammatory agent, antigout or antihyperuricemic agent, nonsteroidal anti-inflammatory agent, steroidal anti-inflammatory agent, anticancer agent, hypnotic or analgesic agent, anxiolytic agent, antipsychotic agent, antidepressant agent, antimanic agent, antihistaminic agent, antiepileptic agent, local anesthetic, antiparkinson agent, muscle relaxant, autonomic or antispasmodic agent, antidiaphoretic agent, cerebral circulation or metabolism-improving drug, cardiac stimulant, antianginal agent, antiarrhythmic agent, antihypertensive agent, vasodilator, decongestant, capillary stabilizer, antihyperlipemic agent, vasopressor, respiratory stimulant, bronchodilator or antiasthmatic agent, antitussive agent, expectorant, therapeutic agent for peptic ulcer, deglutitory agent, antiemetic agent, obstipant or antiflatulent, choleretic agent, antidiabetic agent, pituitary hormone preparation, adrenocortical hormone preparation, sex hormone preparation, prostaglandin, therapeutic agent for thyroid dysfunction, anti-osteoporosis or bone metabolism improving drug, amino acid, vitamin preparation, antithrombotic agent, antibiotic, sulfa drug, antimycotic agent, antiviral agent, anti-HIV agent, antiparasitic or antiprotozoal agent, antirheumatic drug, immunosuppressant, antiallergic agent, peptide hormone preparation, anti-smoking aid, diet ingredient, polysaccharide, plant extract or essential oil, enzyme, and vaccine.

**6.** The transdermal preparation of any one of claims 1 to 5, wherein a dosage form of the preparation is a cream, ointment, liquid, tape, patch, or cataplasm.

**7.** A transmucosal preparation comprising an ingredient to be absorbed transmucosally and a hydroxyapatite.

**8.** The transmucosal preparation of claim 7, wherein the hydroxyapatite has a maximum particle size of 1 µm or less.

**9.** The transmucosal preparation of claim 7, wherein the hydroxyapatite has a maximum particle size of 0.1 µm or less.

**10.** The transmucosal preparation of any one of claims 7 to 9, wherein the hydroxyapatite has a content of 0.1% to 1000% based on a drug to be combined.

**11.** The transmucosal preparation of any one of claims 7 to 10, wherein the ingredient to be absorbed transmucosally comprises one or more selected from the group consisting of: an analgesic, antipyretic, and anti-inflammatory agent, antigout or antihyperuricemic agent, nonsteroidal anti-inflammatory agent, steroidal anti-inflammatory agent, wound healing agent, anticancer agent, hypnotic or analgesic agent, anxiolytic agent, antipsychotic agent, antidepressant agent, antimanic agent, antihistaminic agent, antiepileptic agent, local anesthetic, antiparkinson agent, muscle relaxant, autonomic or antispasmodic agent, antidiaphoretic agent, cerebral circulation or metabolism-improving drug, cardiac stimulant, antianginal agent, antiarrhythmic agent, antihypertensive agent, vasodilator, decongestant, capillary stabilizer, antihyperlipemic agent, vasopressor, respiratory stimulant, bronchodilator or antiasthmatic agent, antitussive agent, expectorant, therapeutic agent for peptic ulcer, deglutitory agent, antiemetic agent, obstipant or antiflatulent, choleretic agent, antidiabetic agent, pituitary hormone preparation, adrenocortical hormone preparation, sex hormone preparation, prostaglandin, therapeutic agent for thyroid dysfunction, anti-osteoporosis or bone metabolism improving drug, amino acids vitamin preparation, hemostatic agent, antithrombotic agent, antibiotic, sulfa drug, antimycotic agent, antiviral agents, anti-HIV agents, antiparasitic or antiprotozoal agents, antirheumatic drugs, immunosuppressant, sunscreen agent, antiallergic agent, ophthalmic preparation, antipsoriatic agent, skin softener or emollient, baldness-remedy or hair growth stimulant, peptide hormone preparation, anti-smoking aid, diet ingredient, polysaccharide, plant extract or essential oil, enzyme, and vaccine.

**12.** The transmucosal preparation of any one of claims 7 to 11, wherein a dosage form of the preparation is a cream, ointment, liquid, tape, patch, cataplasm, suppository, nasal drop, eye drop, vaginal preparation, dentifrice, or chewing gum.
